**Europäisches Patentamt**

(19) **European Patent Office**

**Office européen des brevets**

(11) Veröffentlichungsnummer: **0 046 564**
B1

(12) # EUROPÄISCHE PATENTSCHRIFT

(45) Veröffentlichungstag der Patentschrift:
**21.12.83**

(51) Int. Cl.³: **C 07 C 27/22**, C 07 C 29/16,
C 07 C 45/50

(21) Anmeldenummer: **81106395.7**

(22) Anmeldetag: **18.08.81**

(54) **Verfahren zum Entmetallisieren von Primärprodukten der Oxo-Synthese.**

(30) Priorität: **27.08.80 DE 3032252**

(43) Veröffentlichungstag der Anmeldung:
**03.03.82 Patentblatt 82/9**

(45) Bekanntmachung des Hinweises auf die Patenterteilung:
**21.12.83 Patentblatt 83/51**

(84) Benannte Vertragsstaaten:
**FR GB IT NL**

(56) Entgegenhaltungen:
**DE - B - 1 064 047
DE - B - 2 245 565
US - A - 2 744 921
US - A - 3 361 829**

(73) Patentinhaber: **Ruhrchemie Aktiengesellschaft,
Bruchstrasse 219, D-4200 Oberhausen 13 (DE)**

(72) Erfinder: **Meis, Josef, Nikolaus-Gross-Strasse 9,
D-4200 Oberhausen 12 (DE)**
Erfinder: **Schmidt, Volkmar, Dipl.-Ing., Lützowstrasse 51,
D-4200 Oberhausen 13 (DE)**
Erfinder: **Tummes, Hans, Dr., Dipl.-Chem.,
Oranienstrasse 88, D-4200 Oberhausen 14 (DE)**
Erfinder: **Much, Joachim, Dipl.-Ing., Im Torfveen 2,
D-4200 Oberhausen 14 (DE)**

(74) Vertreter: **Reichelt, Karl-Heinz, Dr., m. Br. Ruhrchemie
Aktiengesellschaft Abt. PLD Postfach 13 01 60,
D-4200 Oberhausen 13 (DE)**

## Verfahren zum Entmetallisieren von Primärprodukten der Oxo-Synthese

Die vorliegende Erfindung betrifft ein Verfahren zum Entmetallisieren von Primärprodukten der Oxosynthese mit Wasserdampf.

Bei den bekannten technischen Oxo-Verfahren werden Olefine mit Kohlenmonoxid und Wasserstoff in Gegenwart von Kobaltverbindungen bei erhöhter Temperatur und erhöhtem Druck umgesetzt. Die hierbei anfallenden, hauptsächlich aus primären Aldehyden und Alkoholen bestehenden Produkte (»Primärprodukte«) enthalten das als Katalysator eingesetzte Kobalt gelöst in Form von Kobaltcarbonylverbindungen. Diese Kobaltcarbonyle erschweren die Weiterverarbeitung der Primärprodukte — z. B. deren Destillation und Hydrierung — so sehr, daß sie aus wirtschaftlichen Gründen in einem besonderen Arbeitsschritt (»Entkobaltung«) entfernt werden müssen.

Die Wirtschaftlichkeit technischer Oxo-Verfahren hängt entscheidend davon ab, die Kobaltcarbonylzersetzung so durchzuführen, daß das anfallende Kobalt bzw. die anfallenden Kobaltverbindungen leicht von den organischen Reaktionsprodukten abgetrennt und ohne großen Aufwand wieder in die Hydroformylierungsstufe zurückgeführt werden können.

Von den zahlreichen bekannten Verfahren zur Entkobaltung der primären Oxo-Produkte zeichnet sich die Anwendung von Wasserdampf durch Wirtschaftlichkeit und den Vorteil, daß die in den Oxorohprodukten enthaltenen höheren Kondensationsprodukte gleichzeitig teilweise gespalten werden, besonders aus. Je nach den angewandten Reaktionsbedingungen, vor allem in Abhängigkeit von den Schwefelgehalten im Reaktionssystem, werden bei der Wasserdampfbehandlung aus den Kobaltcarbonylverbindungen unterschiedlich Zersetzungsprodukte erhalten, nämlich Kobaltmetall oder bevorzugt Kobalthydroxid oder — besonders in Gegenwart von Säuren — Kobaltsalze dieser Säuren.

Aus der FR-PS 1 018 055 ist es bekannt, die im Primärprodukt der Oxosynthese enthaltenen Kobaltcarbonylverbindungen mit Wasserdampf unter Bildung feinverteilten Kobaltmetalls zu zersetzen. Nach Kühlung des Gemisches und Abzug der Gase über Kopf läßt sich das Metall dann durch mechanische Trennvorrichtungen von der organischen, die Produkte der Oxosynthese erhaltenen Phase abtrennen.

Bei der Durchführung dieses Verfahrens hat sich herausgestellt, daß in den meisten Fällen nur ein Teil des Kobalts als Metall abgeschieden wird, während der Rest in gelöster Form in der wäßrigen Phase zu finden ist. Überdies stellt diese bekannte Arbeitsweise nicht sicher, daß das Kobaltmetall stets in leicht abtrennbarer, z. B. filtrierbarer Beschaffenheit erhalten wird.

Nach der Lehre der US-PS 2 779 796 können durch Behandlung des Rohproduktes mit Wasserdampf die löslichen Kobaltverbindungen durch Zersetzung oder Fällung soweit abgetrennt werden, daß keine umfangreiche Verschmutzung der nachgeschalteten Apparate auftritt, wenn während der Behandlung des Rohproduktes mit Wasserdampf jede Berührung mit einer feststehenden Heizfläche, deren Temperatur höher als der Siedepunkt der Wasser-Produkt-Mischung ist, vermieden wird. Die Behandlung des Rohproduktes kann bei diesem Prozeß außer in einem Rührkessel alternativ auch dadurch erfolgen, daß die Ströme von Dampf und sauerstoffhaltigem Rohprodukt in einem turbulenten Strömungsfeld, wie es z. B. in einem Mischrohr gegeben ist, zusammengemischt werden. Dieses Verfahren ist jedoch nur bei Oxorohprodukten mit sehr geringen Co-Gehalten von 0,1% und weniger anwendbar. Derart geringe Co-Einsätze in der Oxosynthese erfordern übergroße Reaktionsvolumen und hohe Hydroformylierungstemperaturen und sind daher besonders für niedrige Olefine wirtschaftlich ungeeignet. Außerdem werden trotz des geringen Co-Gehaltes nur etwa 90% Co entfernt. Bei den in der Technik gebräuchlichen Co-Einsätzen würde sich mit diesem Verfahren Co metallisch fest an Rührern und Mischrohren absetzen.

Die analoge Mischung von Oxorohprodukt mit Heißwasser in einem Rohr ist in der DE-PS 1 024 499 beschrieben. Aber auch bei diesem Prozeß ist der Entkobaltungsgrad unbefriedigend, und im gesamten System sowie in den Nachverarbeitungsapparaten kommt es zu starken Verlegungen.

In der DE-AS 2 245 565 ist ein Verfahren zur möglichst vollständigen Abscheidung des Kobalts durch Behandlung des Primärprodukts der Oxo-Synthese mit Wasserdampf in Gegenwart von Schwefel oder Schwefelverbindungen beschrieben. Angaben über die Art und Weise, in der Kobalt enthaltendes Produkt und Wasserdampf zusammengeführt werden, sind dieser Druckschrift nicht zu entnehmen. Obgleich eine nahezu vollständige Abscheidung des Kobalts nach dieser Arbeitsweise erreicht wird, ist nicht sichergestellt, daß Verlegungen in der Apparatur vermieden werden.

Nach dem Verfahren der US-PS 2 744 921 erfolgt die Abtrennung des Kobalts aus dem Primärprodukt der Oxo-Synthese in einem 2stufigen Prozeß, wobei das Rohprodukt zunächst mit der wäßrigen Lösung einer organischen Säure extrahiert wird. Aus dieser wäßrigen Lösung muß das Kobalt in einer weiteren Verfahrensstufe isoliert werden. Dieser Prozeß ist apparativ sehr aufwendig und erfordert darüber hinaus den Einsatz zusätzlicher Chemikalien.

Auch in der US-PS 3 361 829 wird die extraktive Abtrennung von Kobaltverbindungen aus dem Hydroformylierungsprodukt beschrieben. Als Extraktionsmittel dient hierbei die wäßrige Lösung geradkettiger Alkohole. Der nach diesem Verfahren erzielbare Kobalt-Restgehalt im Oxo-Produkt ist für viele Anwendungsgebiete zu hoch.

Gemäß dem in der DE-AS 1 064 047 beschrie-benen Verfahren werden Kobaltcarbonylverbin-dungen aus den Anlagerungsprodukten von Kohlendioxid und Wasserstoff an Olefine durch Zersetzung mit Wasser bei 120 bis 260°C entfernt. Zu diesem Zweck wird das Primärprodukt der Oxo-Synthese durch ein mit Wasser praktisch völlig angefülltes beheiztes Katalysatorzersetzungsgefäß geführt. Auch nach dieser Arbeitsweise kann eine Verlegung von Anlageteilen nicht vermieden werden.

Es bestand somit die Aufgabe, einen Prozeß zu entwickeln, der die aufgezeigten Nachteile vermeidet und eine vollständige Abtrennung des Kobalts in solcher Form gewährleistet, die einen direkten Wiedereinsatz in die Hydroformylierung gestattet.

Erfindungsgemäß wird zur Zersetzung der im primären Reaktionsprodukt der Oxosynthese gelösten Kobaltcarbonylverbindungen zu festen kobalthaltigen Produkten, vorwiegend Kobaltmetall, durch innige Vermischung bei 100 bis 200°C und 5 bis 25 bar in einem zylindrischen Gefäß ohne Einbauten und unmittelbarem Wiedereinsatz der aus den Reaktionsprodukten abgetrennten Kobaltprodukte in die Oxosynthese derart gearbeitet, daß das zylindrische Reaktionsgefäß einen Konusboden besitzt, der Dampf kontinuierlich von unten durch eine gekühlte Düse in 1/2 bis 2/3 Höhe vom Konusboden eingeführt wird, dort nach oben mit einer Geschwindigkeit von 50 bis 150, bevorzugt 80 bis 120 m/s koaxial zum zylindrischen Reaktionsgefäß ausströmt, dabei unmittelbar nach dem Austritt aus der Düse auf das in den Strömungskegel des Dampfes eingeleitete kobaltcarbonylhaltige Oxo-Produkt trifft, wobei der Flüssigkeitszustand durch laufende Abnahme des Reaktionsproduktes am tiefsten Punkt des Konusbodens im zylindrischen Reaktionsgefäß auf einer Höhe gehalten wird, die dem 1- bis 2fachen des Durchmessers des zylindrischen Reaktionsgefäßes entspricht.

Die vollständige Zersetzung der Metallcarbo-nylverbindungen ist nicht nur eine Forderung für eine störungsfreie Aufarbeitung des entmetallisierten Oxoproduktes, sondern auch eine Voraussetzung dafür, daß sich kein Co-Metall im Entmetallisierungssystem abscheidet.

Daher wird die vollständige Zersetzung der Kobaltcarbonyle, die durch geringe Mengen anderer Metallcarbonyle, z. B. des Eisens, verunreinigt sein können, erfindungsgemäß unter Bedingungen durchgeführt, die sicherstellen, daß bei der Durchmischung von Primärprodukt und Dampf Kobaltteilchen in solcher Form und Größe nicht erhalten werden, die in Bewegung bleiben und sich nicht abscheiden. Wichtig ist, daß der Zersetzungsraum eine möglichst geringe Oberfläche aufweist und außer den Vorrichtungen zur Primärprodukt- und Dampfzugabe keinerlei Einbauten besitzt.

Eine ausreichend gute Bewertung des Primärprodukts, die das Absetzen des durch Zersetzung entstandenen Kobalts verhindert, wird erreicht durch die Teilentspannung des im Primärprodukt noch gelösten Syntheserestgases und durch Einblasen von Wasserdampf mit einer Geschwindigkeit von 50 bis 150 m/s, am besten 80 bis 120 m/s, zentral am Boden des Zersetzungsgefäßes kurz über dem Bodenablauf.

Die Zugabe des Primärproduktes erfolgt diametral zum Dampfeinsatz von oben, wobei der Einlaufstutzen nur leicht in die flüssige Phase eintaucht oder von unten unmittelbar neben dem Dampfeintritt.

Um das durch Zersetzung entstandene Kobalt vollständig in Bewegung zu halten und um die zusätzliche Entstehung von höhersiedenden Nebenprodukten zu vermeiden, stellt man die Verweilzeit des Primärproduktes im Zersetzungsraum auf 4 bis 15 Minuten, bei Aldehyden mit 3 bis 5 C-Atomen vorzugsweise auf 6 bis 10 Minuten, ein.

Es ist erforderlich, die Temperatur im Zersetzungsraum mit steigendem Kobaltgehalt sowie mit zunehmender C-Zahl des Primärproduktes anzuheben. Die Änderung des Kobaltgehalts in dem beim vorliegenden Verfahren interessanten Bereich führt nur zu einer verhältnismäßig geringen Verschiebung der Zersetzungstemperatur. Eine Erhöhung des Kobaltgehalts im Primärprodukt von 0,6 Gew.-% auf 1,0 Gew.-% hat nur eine Anhebung der Temperatur im Zersetzungsraum um 2 bis 3°C zur Folge. In einem etwas weiteren Rahmen wird die Zersetzungstemperatur durch die C-Zahl des Primär-produktes beeinflußt. So werden beispielswei-se, in der gleichen Entmetallisierungseinrichtung bei einem konstanten Kobaltgehalt des Primärproduktes von ca. 0,6 Gew.-% für das Primärprodukt aus Propylen Temperaturen im Zersetzungsraum zwischen 145°C und 155°C, für das Primärprodukt aus Dicyclopentadien Temperaturen zwischen 150°C und 155°C und für das Primärprodukt aus Tetrapropylen 155°C bis 160°C benötigt.

Der Druck im Zersetzungsraum wird, um eine optimale Entkobaltung zu erzielen, der benötigten Zersetzungstemperatur entsprechend nach dem Verfahren der Anmeldung so eingestellt, daß etwa 15 bis 45% des Primärproduktes mit dem im Primärprodukt gelösten Abgas in Dampfform am Kof des Zersetzungsgefäßes anfallen.

Die Abnahme der Hauptmenge des entmetalli-sierten Primärproduktes, das etwas Wasser-dampf-Kondensat mit geringen Anteilen darin gelöstem Kobalt mitführt, erfolgt am Boden des Zersetzungsgeäßes. Der Boden ist zweckmäßigerweise als Konus ausgebildet, um Co-Ablagerungen zu vermeiden.

Bei den bisher bekannten, nicht mit Säure arbeitenden Entkobaltungsverfahren besteht die Gefahr, daß die Abgas-Dämpfe-Leitung durch mitgerissenen Kobaltstaub verlegt wird. Solche Verlegungen werden bei dem erfindungsgemäßen Verfahren vermieden, wenn der Gasraum über den die Zersetzungszone bildenden Flüssigkeitsraum wenigstens so groß ist wie dieser. Zweckmäßig ist der Gasraum 1½mal so groß wie der Flüssigkeitsraum. Wird mit unzureichendem Gasraum gearbeitet, muß die Abgas-Dämpfeleitung möglichst bis zum Kühlereintritt gekühlt werden.

Bei der Durchführung des erfindungsgemäßen Verfahrens hat es sich besonders bei Zersetzungsgefäßen mit großen Durchmessern als günstig erwiesen, Primärprodukt und Wasserdampf mittels der in F i g. 2 dargestellten und im folgenden näher erläuterten Vorrichtung in das Reaktionsgefäß einzuführen. Bei dieser Vorrichtung handelt es sich um eine kombinierte Düse für Primärprodukt und Wasserdampf, die mit Wasser gekühlt wird. Sie besteht aus einem Kernrohr 11, durch welches das Reaktionsprodukt eingeleitet wird, einem Ringraum 12 und das Kernrohr oder aus Rohren, die ringförmig um das Kernrohr angeordnet sind, durch welche der Wasserdampf eingeführt wird. Um eine Wärmeübertragung vom Wasserdampf zum Primärprodukt zu vermeiden, ist das Kernrohr 11 vom Ringraum 12 bzw. den ringförmig angeordneten Rohren durch einen mit einem Kühlmedium beaufschlagten Raum 13 getrennt.

### Beispiel 1

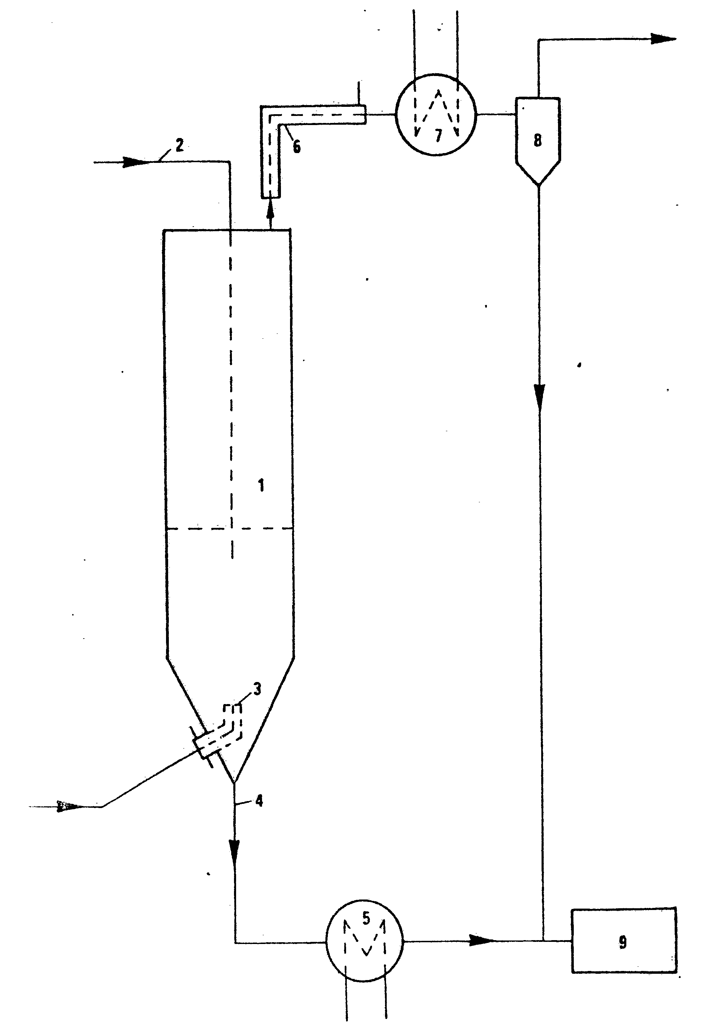

### (Siehe hierzu das in Fig. 1 gezeigte Verfahrensschema)

In ein mit einem Konusboden ausgerüstetes, zylindrisches Gefäß (Reaktionsgefäß 1) wird zentral von oben über eine Leitung 2, die in eine Flüssigkeitssäule eintaucht direkt unmittelbar aus einem Hochdruckabscheider der Hydroformylierung kommendes Primärprodukt, das 3 bis 6 g Co/l in Form gelöster Carbonylverbindungen enthält, entspannt.

Kurz oberhalb des Konusablaufs wird Wasserdampf von 20 bis 40 bar durch eine Düse 3, deren Austrittsrichtung senkrecht nach oben auf den Primärprodukteintritt gerichtet ist, mit einer Geschwindigkeit von etwa 100 m/s eingeblasen. Die Zersetzungstemperatur liegt zwischen 150 und 160°C, während ein Druck von 17 bis 19 bar eingehalten wird.

An einem Konusablauf 4 werden das gesamte Kobalt, 70% des eingesetzten Wasserdampfs als heterogene Wasserphase, die etwas Kobalt enthält und etwa 70% des entmetallisierten Primärproduktes über einen Kühler 5 abgenommen. Über Kopf des Zersetzungsgefäßes gehen der nicht kondensierte Wasserdampf, etwa 30% entmetallisiertes Primärprodukt sowie das Restgas, das im Primärprodukt der Oxosynthese gelöst war, über eine Leitung 6 ab.

Nach Durchströmen einer Kühlung 7 und einer Gasabscheidung 8 werden die Flüssigprodukte wieder vereint und einer Phasentrennung 9 zugeführt. Das Primärprodukt enthält Zersetzung der Carbonyle und der Abtrennung des festen Kobalts weniger als 0,1% des ursprünglichen Co-Gehaltes. Der abgetrennte Kobaltschlamm wird unmittelbar wieder in die Hydroformylierung eingesetzt.

Aus der wäßrigen Lösung kann das wenige restliche Kobalt z. B. durch Fällung mit Alkalihydroxid für den Widereinsatz in die Synthese zurückgewonnen werden.

### Beispiel 2

Als Zersetzungsgefäß dient ein zylindrischer Behälter mit konischem Boden.

Aus Diisobutylen hergestelltes Primärprodukt, das direkt aus der Oxosynthese kommt, wird — in der gleichen Weise wie in Beispiel 1 beschrieben — in das Zersetzungsgefäß eingegeben. Gleichzeitig wird Wasserdampf (25 bis 30 bar) durch eine Düse zugeführt. Die Zersetzung der Carbonyle erfolgt bei 160 bis 170°C und etwa 16 bar. Das entkobaltete, wasserklare Produkt enthält etwa 5 mg Co/l.

### Beispiel 3

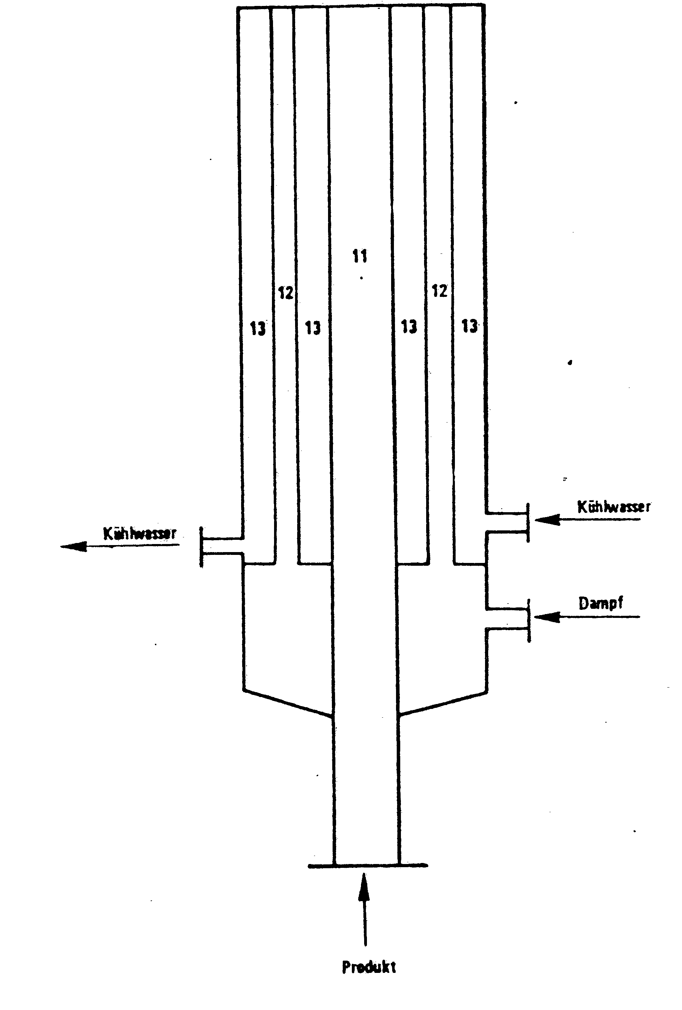

In einem zylindrischen Gefäß befindet sich in einem konusförmigen Boden eine kombinierte, wassergekühlte Eingabevorrichtung, wie in Fig. 2 dargestellt.

Durch ein Kernrohr 11 wird Primärprodukt aus der Hydroformylierung von Propylen eingeleitet, während durch mehrere Dampfdüsenrohre 12 Wasserdampf von 28 bar eingedrückt wird. Die Eingabekombination ist vom Kühlmantel 13 umschlossen. Die Zersetzung erfolgt bei einer Temperatur von 150 bis 160°C und einem Druck von etwa 19 bar. Das Primärprodukt enthält nach der Entkobaltung 3 mg Co/l.

**Patentanspruch**

Verfahren zur Zersetzung der im primären Reaktionsprodukt der Oxo-Synthese gelösten Kobaltcarbonylverbindungen zu festen kobalthaltigen Produkten, vorwiegend Kobaltmetall, durch innige Vermischung des primären Reaktionsproduktes mit Wasserdampf bei 100 bis 200° und 5 bis 25 bar in einem zylindrischen Gefäß ohne Einbauten und unmittelbarem Wiedereinsatz der aus den Reaktionsprodukten abgetrennten Kobaltprodukte in die Oxo-Synthese, dadurch gekennzeichnet, daß das zylindrische Reaktionsgefäß einen Konusboden besitzt, der Dampf kontinuierlich von unten durch eine gekühlte Düse in 1/2 bis 2/3 Höhe vom Konusboden eingeführt wird, dort nach oben mit einer Geschwindigkeit von 50 bis 150, bevorzugt 80 bis 120 m/s koaxial zum zylindrischen Reaktionsgefäß ausströmt, dabei unmittelbar nach dem Austritt aus der Düse auf das in den Strömungskegel des Dampfes eingeleitete kobaltcarbonylhaltige Oxo-Produkt trifft, wobei der Flüssigkeitsstand durch laufende Abnahme des Reaktionsproduktes am tiefsten Punkt des Konusbodens im zylindrischen Reaktionsgefäß auf einer Höhe gehalten wird, die dem 1- bis 2fachen des Durchmessers des zylindrischen Reaktionsgefäßes entspricht.

## Claim

Process for decomposing the cobalt carbonyl compound dissolved in the primary reaction product of the oxo-synthesis into a solid, cobalt-containing product, substantially cobalt metal, by intimately mixing the primary reaction product with steam at 100 to 200°C and at 5 to 25 bar in a cylindrical vessel without built-in fitments and directly reusing the cobalt products separated from the reaction products in the oxo-synthesis, characterized in that the cylindrical reaction vessel has a conical base, the steam is fed continuously from below into the reaction vessel through a colled nozzle located at 1/2 to 2/3 the height of the conical base, thence flows upwards at a rate of 50 to 150 m/s, preferably 80 to 120 m/s, coaxially to the cylindrical reaction vessel and thereby comes into contact with the cobalt carbonyl-containing oxo-product immediately after exiting from the cooled nozzle, the cobalt-carbonyl-containing oxo-product being introduced into the flow cone of the steam, the liquid level being maintained at a height corresponding to 1 to 2 times the diameter of the cylindrical reaction vessel by continuously removing the teaction product at the lowest point of the conical base in the cylindrical reaction vessel.

## Revendication

Procédé pour décomposer les composés de cobalt-carbonyle dissous dans le produit de réaction primaire de la synthèse Oxo en produits solides contenant du caobalt, principalement du cobalt métallique, par mélange intime du produit de réaction primaire avec de la vapeur d'eau à une température de 100 à 200°C et une pression de 5 à 25 bars dans un récipient cylindrique sans dispositifs intérieurs, et réutiliser directement les produits à base de cobalt séparés des produits de réaction dans la synthèse Oxo, caractérisé en ce que le récipient de réaction cylindrique a un fond conique, la vapeur d'eau est introduite en continu par le bas au travers d'une tuyère refroidie à une hauteur représentant 1/2 à 2/3 de la hauteur du fond conique, elle s'écoule ensuite vers le haut à une vitesse de 50 à 150, de préférence de 80 à 120 m/s, en direction co-axiale avec le récipient de réaction cylindrique, entre en contact directement après sortie de la tuyère avec le produit Oxo contenant des composés de cobalt-carbonyle injecté dans le cône d'écoulement de la vapeur d'eau, le niveau de liquide étant maintenu par évacuation continue du produit de réaction au point le plus bas du fond conique du récipient de réaction cylindrique à une hauteur correspondant à une à deux fois le diamètre du récipient de réaction cylindrique.

Kühlwasser

Kühlwasser

Dampf

Produkt